(19) **Europäisches Patentamt / European Patent Office / Office européen des brevets**

(11) **EP 2 856 923 A1**

(12) **EUROPEAN PATENT APPLICATION**
published in accordance with Art. 153(4) EPC

(43) Date of publication:
08.04.2015 Bulletin 2015/15

(51) Int Cl.:
**A61B 1/00** (2006.01)  **A61B 1/04** (2006.01)

(21) Application number: 13797833.4

(22) Date of filing: 16.02.2013

(86) International application number:
**PCT/JP2013/053772**

(87) International publication number:
**WO 2013/179693 (05.12.2013 Gazette 2013/49)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **29.05.2012 JP 2012122489**

(71) Applicant: **Tokyo Institute of Technology Tokyo 152-8550 (JP)**

(72) Inventors:
• **TADANO, Kotaro**
**Tokyo 152 8550 (JP)**
• **KAWASHIMA, Kenji**
**Tokyo 152 8550 (JP)**

(74) Representative: **Piésold, Alexander James Dehns**
**St Bride's House**
**10 Salisbury Square**
**London**
**EC4Y 8JD (GB)**

(54) **ENDOSCOPE OPERATION SYSTEM**

(57) The objective is to control a movement velocity of an endoscope in a body in response to an amount of insertion of the endoscope in view of safety without influence of a strong magnet. A velocity-control computation section 48 adjusts a velocity of an imager to be changeable in response to an amount of insertion of the imager of the endoscope in a body by computing a target velocity vector Vxy. Thereby, increasing of the insertion amount of the imager of the endoscope inserted in the body in a movement direction makes a target velocity vector V'xy of the imager of the endoscope larger. Decreasing of the insertion amount of the imager of the endoscope, or pulling the imager of the endoscope out of the body, makes a target velocity vector V'xy of the imager of the endoscope smaller.

FIG. 1

**Description**

TECHNICAL FIELD

The present invention relates to an endoscope operation system.

BACKGROUND ART

A surgery includes an endoscopic surgery, which is widely performed in place of laparotomy, due to advantages such as a shorter postoperative recovery time and a smaller surgical cut. Such an endoscopic surgery has a proposed master-slave endoscope operation system capable of remote control. Such an endoscope operation system, as shown, for example, in Patent Document 1, includes an endoscope with a zoom lens whose magnification is controlled based on a detection output from a posture sensor provided in a head mount display (also called an HMD hereinbelow) to detect movement of the head of an operator. Movement of the head of the operator is retrieved as a displacement of the attitude sensor relative to a magnetic source producing a magnetic field. Thus, for example, if the operator moves to the left relative to the patient, the endoscope obtains imaged data obtained through a solid-state image sensor, and the imaged data is used to display a left image on a pair of liquid crystal monitors inside the HMD. When the operator moves toward the patient, the operator obtains a field of view magnified by the zoom lens. Hence, the operator can three-dimensionally observe the inside of a cavity of the body into which the endoscope is inserted.

In addition, there has been conventionally proposed an endoscope hold device including a five-bar linkage mechanism, a ball joint portion for retaining a trocar penetrating an abdominal wall at the abdominal wall portion, and a drive portion and an operation unit configured to drive the linkage mechanism. In such a configuration, a laparoscope being a type of an endoscope rapidly switches the display distance by use of zooming, and a controller switch allows the zooming laparoscope to move speedily to a position desired by the operator.

PRIOR ART DOCUMENT

PATENT DOCUMENT

Patent Document 1: Japanese Patent Application Publication No. Hei 10-309258

SUMMARY OF THE INVENTION

PROBLEM TO BE SOLVED BY THE INVENTION

It may be difficult to install the endoscope operation system under an environment where a magnetic resonance imaging (MRI) apparatus is installed because the aforementioned magnetic source for producing a magnetic field may be influenced by the strong magnet in the magnetic resonance imaging apparatus. In addition, it is desired from a safety perspective that, in moving the laparoscope speedily, the above-described endoscope support device should control the velocity of moving the laparoscope inside the body in response to the amount of insertion of the laparoscope into the body. However, such a device is yet to be developed.

In consideration of the above problems, the present invention has an objective of providing an endoscope operation system capable of controlling, from a safety perspective, the velocity of moving an endoscope inside a body in response to the amount of insertion of the endoscope, without the influence of a strong magnet.

MEANS FOR SOLVING THE PROBLEM

To achieve the above objective, an endoscope operation system according to the present invention includes: a hold arm unit reciprocatably and rotatably supported and configured to send attitude information thereof; an endoscope supported by the hold arm unit and including an imager at a distal end thereof; a display unit configured to display an image by a head mount display based on an image signal from the imager of the endoscope; a posture detector including a sensor configured to detect a postural state of an operator, and a foot switch. The distal end of the endoscope has a movement direction and a velocity to be controlled based on postural movement information from the posture detector, attitudinal information on the hold arm unit, and camera information on the imager of the endoscope.

ADVANTAGEOUS EFFECTS OF THE INVENTION

In the endoscope operation system according to the present invention, the movement direction and velocity of the distal end of the endoscope are controlled based on the postural motion information from the posture detector, the attitudinal information on the hold arm unit, and the camera information on the imager of the endoscope. Thus, from a safety perspective, the movement velocity of the endoscope inside the body is controlled in response to the insertion amount of the endoscope. Moreover, for example, driving the hold arm unit by multiple pneumatic actuators provides an advantageous effect that interference with a magnetic field is less likely to occur, which enables the endoscope operation system not to be influenced by a strong magnet.

BRIEF DESCRIPTION OF THE DRAWINGS

Fig. 1 is a block diagram showing an overall configuration of an example of an endoscope operation system according to the present invention.
Fig. 2 is a diagram schematically showing the overall configuration of the endoscope operation system according to the present invention, together with an operator.
Fig. 3 is a diagram showing a hold arm unit used in an example of the endoscope operation system according to the present invention.
Fig. 4 is a characteristic diagram showing an effect of drift compensation on an output from a gyro sensor in an example of the endoscope operation system according to the present invention.
Fig. 5 is a flowchart showing, in a case where a drift-compensation computation section in an example of the endoscope operation system according to the present invention is configured of a microcomputer, a program executed by the microcomputer.

MODES FOR CARRYING OUT THE INVENTION

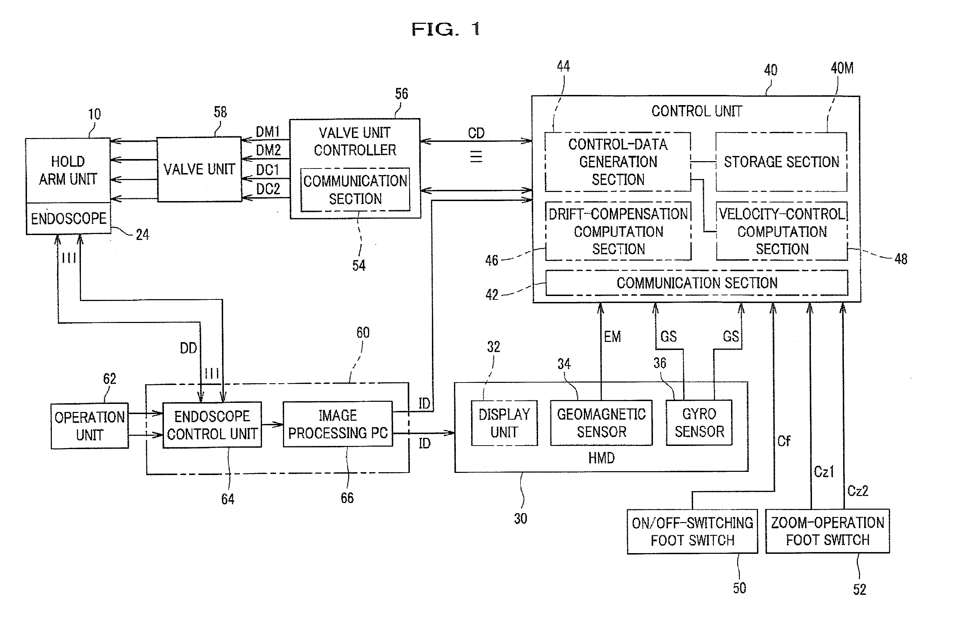

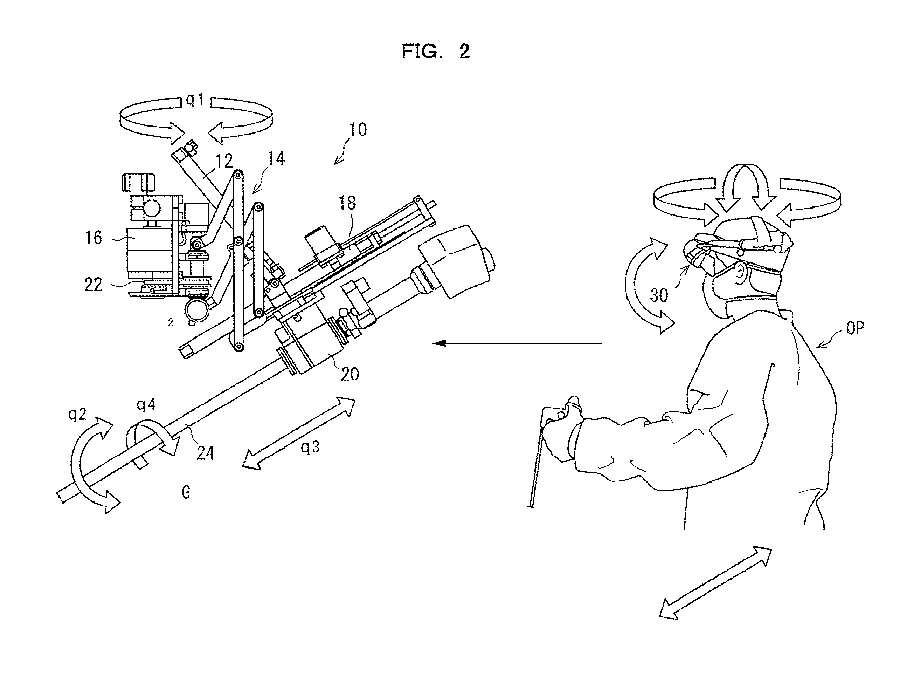

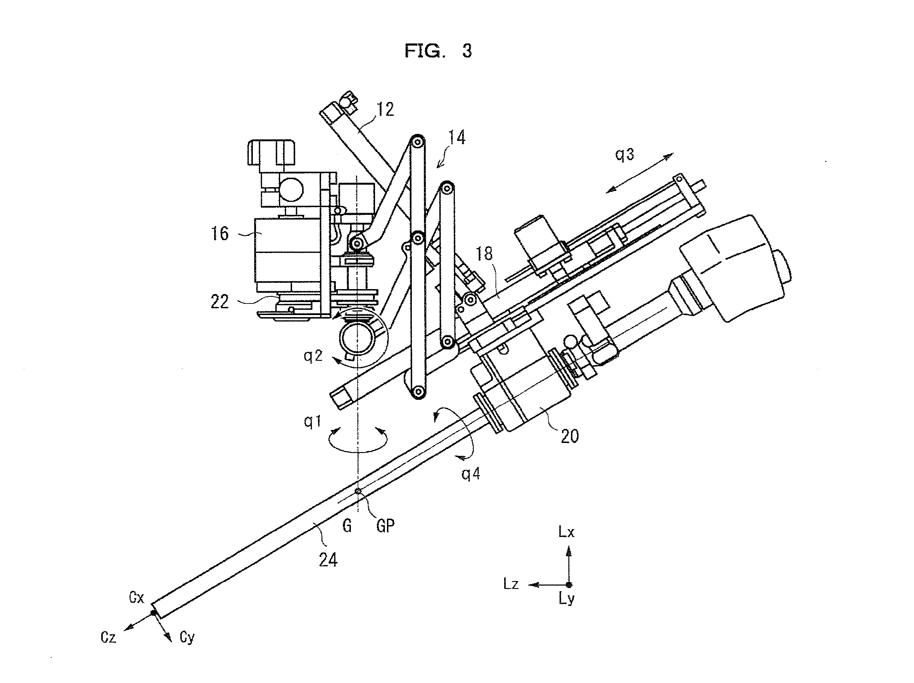

Fig. 2 shows the configuration of an example of an endoscope operation system according to the present invention, together with an operator.
In Fig. 2, the endoscope operation system is configured including, as its main components, an endoscope 24, a hold arm unit 10 configured to hold the endoscope 24 and control the attitude of the endoscope 24, and a head mount display 30 (also called an HMD 30 hereinbelow) detachably fitted to the head of an operator OP.
The endoscope 24 is, for example, configured including a flexible inserted portion having an imager at its distal end which is to be inserted into the body, an operation unit 62 (see Fig. 1) configured to control an optical system, and a connection unit connected to the operation unit to connect a light source or the like to the operation unit.
The imager includes an optical portion of an objective lens and the like, a solid-state image sensor, and a zoom mechanism including an actuator configured to control a lens of the optical unit to magnify or reduce the size of an image obtained by the imager. The zoom mechanism of the imager is controlled by an endoscope control unit 64 to be described later. There is a light guide provided next to the objective lens at the distal end of the inserted portion. The light guide is configured to illuminate the inside of the body with light led from the aforementioned light source.
The endoscope may employ a rigid endoscope or a flexible endoscope.
The HMD 30 is, as shown in Fig. 2, fitted to the head of the operator OP. The HMD 30 includes paired left and right display units 32 (see Fig. 1) at positions facing the front of the face of the operator OP and corresponding to the respective eyes of the operator OP. The display units 32 are configured to display, for example, color images in 3D format. Note that the display units are not limited to such an example, and may be configured to display, for example, black-and-white images in 2D format.
The entire HMD 30 follows the movement of the head of the operator OP. Thus, the HMD 30 is, as shown by the arrows in Fig. 2, capable of the following motions from a viewpoint of the operator OP: rightward (clockwise) rotation with the neck being the center axis (rightward turning), leftward (counterclockwise) rotation with the neck being the center axis (leftward turning), rotation in longitudinal directions of the neck (bending, extension), tilt to the right of the neck (rightward bending), and tilt to the left of the neck (leftward bending).
The HMD 30 includes a gyro sensor (also called a gyroscope hereinbelow) 36 configured to detect the turning, lateral bending, bending, and extension, and a geomagnetic sensor 34 (see Fig. 1). Detection outputs from the gyro sensor 36 and the geomagnetic sensor 34 are supplied to a control unit 40 to be described later. The geomagnetic sensor 34 may be replaced with an acceleration sensor.
The hold arm unit 10 is supported by a mounting (not shown) located adjacent to an operating table which is away from the operator, with an intervening bracket (not shown) of a vane motor unit 16 to be described later. As shown in Figs. 2 and 3, the hold arm unit 10 includes as its main components: a chassis configured to movably support a vane motor 20 which turnably supports the endoscope 24; a pneumatic cylinder 18 fixed to the chassis and configured to move the endoscope 24 and the vane motor 20 toward or away from the patient; the vane motor unit 16 supported with an intervening parallel linkage mechanism 14 whose one end portion is supported by the aforementioned chassis; a rotary shaft configured to turn the entire chassis by being turned with an intervening timing belt pulley connected to an output shaft of the vane motor unit 16 and a timing belt 22; and a pneumatic cylinder 12 configured to drive the parallel linkage mechanism 14.

The parallel linkage mechanism 14 has link members serving as a part thereof. Each link member is connected at one end to the rotary shaft and at the other end to the chassis. Thereby, for example, when the rod of the pneumatic cylinder 12 connected to the parallel linkage mechanism 14 is extended, the chassis turns clockwise about the lower end of the rotary shaft in Fig. 3, and when the rod of the pneumatic cylinder 12 is contracted, the chassis turns counterclockwise about the lower end of the rotary shaft in Fig. 3. In other words, as will be described later, the imager of the endoscope 24 is movable about the rotation center point GP in a direction corresponding to the rotation (bending, extension) of the head of the operator wearing the HMD 30 in the longitudinal direction of the neck of the operator. The rotation center point GP is located, near the body wall of the patient, on a straight line which is common to a rotation axis G, to be described later, of the rotary shaft. The rotation axis G is set to be parallel to an Lx coordinate axis of the orthogonal coordinate system in Fig. 3 for the hold arm unit 10. The Lx coordinate axis is set in a direction orthogonal to the body wall of the patient, and a coordinate axis Lz is set to be perpendicular to the Lx coordinate axis.

The pneumatic cylinder 18 is supported by the chassis such that a rod of the pneumatic cylinder 18 is approximately parallel to the center axis of the endoscope 24. When the rod of the pneumatic cylinder 18 is extended, the imager of the endoscope 24 and the vane motor 20 move relative to the chassis in a direction away from the patient in Fig. 3, and when the rod of the pneumatic cylinder 18 is contracted, the imager of the endoscope 24 and the vane motor 20 move relative to the chassis in a direction toward the patient in Fig. 3.

The link members of the parallel linkage mechanism 14 are connected at their respective one ends to the rotary shaft at positions on the center axis of the rotary shaft away from each other by a predetermined distance, the rotary shaft being provided side by side with the vane motor unit 16. The rotary shaft is supported by the vane motor unit 16 to be turnable about the rotation axis G. Thereby, while the vane motor unit 16 is being operated, the imager of the endoscope 24 and the vane motor 20 are turnable about the rotation axis G. In other words, as will be described later, the imager of the endoscope 24 is movable in a direction corresponding to the turning of the head of the operator wearing the HMD 30 about the neck of the operator.

An area around the operation unit of the endoscope 24 is turnably supported by the vane motor 20. Thereby, the imager of the endoscope 24 rotates (roll) a predetermined angle about the rotation center axis of the vane motor 20. In other words, as will be described later, the imager of the endoscope 24 is moved in a direction corresponding to lateral bending of the operator wearing the HMD 30.

Further, in an example of the endoscope operation system according to the present invention, as shown in Fig. 1, the endoscope operation system includes the control unit 40 configured to control the operation of the hold arm unit 10 and an endoscope control system 60.

The endoscope control system 60 includes: the endoscope control unit 64 configured to control operation of the zoom mechanism portion and the light source of the endoscope 24 based on a set of instruction signals from the operation unit 62; and an image processing PC 66 configured to perform a predetermined image processing based on imaged data DD obtained from the solid-state image sensor of the endoscope 24 through the endoscope control unit 64.

The image processing PC 66 generates image data ID by performing a predetermined image processing based on the imaged data DD and supplies the image data ID to the control unit 40 and the HMD 30. Thereby, an image based on the image data ID from the image processing PC 66 is displayed in 3D (three-dimensional) format on the display units 32 of the HMD 30.

The control unit 40 is supplied with: a set of signals GS from the gyro sensor 36 of the HMD 30, the signals GS representing angular velocity vectors of the head of the operator in the above-described directions; a set of signals EM from each geomagnetic sensor 34, the signals EM representing tilting angles in the respective directions of the head of the operator; an instruction signal Cf from an on/off-switching foot switch 50, the instruction signal Cf representing an instruction to stop the operation of the hold arm unit 10; and an instruction signal Cz1 or an instruction signal Cz2 from a zoom-operation foot switch 52, the instruction signal Cz1 representing an instruction to increase, by a predetermined amount, the amount of insertion of the inserted portion of the endoscope 24 into the body, the instruction signal Cz2 representing an instruction to decrease, by a predetermined amount, the amount of insertion of the inserted portion of the endoscope 24.

The control unit 40 includes a storage section 40M storing: program data for control of the pneumatic pressures for the vane motor unit 16, the vane motor 20, the pneumatic cylinder 12, and the pneumatic cylinder 18; the image data ID from the image processing PC 66; data representing a result of computation by a drift-compensation computation section 46 to be described later; data representing a result of computation by a velocity-control computation section 48; the set of signals EM representing the tilt angles from the geomagnetic sensor 34; and the like.

The control unit 40 has a communication section 42 configured to transmit and receive control data CD to and from a communication section 54 of a valve unit controller 56 bi-directionally. The valve unit controller 56 generates, based on the control data CD from the control unit 40, control signals DM1, DM2, DC1, and DC2 to control the vane motor unit 16, the vane motor 20, the pneumatic cylinder 12, and the pneumatic cylinder 18 of the hold arm unit 10, respectively, and supplies the signals to a valve unit 58. Based on the control signals DM1, DM2, DC1, and DC2, the valve unit 58 controls the respective valves to supply operating air from an air supply source to the vane motor unit 16, the vane motor 20, the pneumatic cylinder 12, and the pneumatic cylinder 18 of the hold arm unit 10.

Although the valve unit controller 56 is provided in the above example, the present invention is not limited to such an example. It goes without saying that, for example, the control unit 40 and the valve unit 58 may be directly wired without the valve unit controller 56 so that the control unit 40 controls the hold arm unit 10.

The control unit 40 has the hold arm unit 10 operate so that the hold arm unit 10 controls the amount of insertion and velocity of the inserted portion of the endoscope 24 into the body of the patient and the attitude of the imager of the endoscope 24. The control unit 40 also performs a drift compensation on the set of signals GS at a predetermined timing, the signals GS representing angular velocity vectors of the head of the operator in the above-described directions from the gyro sensor 36 of the HMD 30.

The velocity-control computation section 48 of the control unit 40 sets a target velocity value $P_{ref}$ overdot of the imager of the endoscope 24 based on: the instruction signal Cz1 or the instruction signal Cz2 from the zoom-operation foot switch 52 of the HMD 30, the instruction signal Cz1 representing an instruction to increase, by a predetermined amount, the amount of insertion of the inserted portion of the endoscope 24 into the body, the instruction signal Cz2 representing an instruction to decrease, by a predetermined amount, the amount of insertion of the inserted portion of the endoscope 24; and the set of signals GS representing angular velocity vectors of the head of the operator in the above-described directions from the gyro sensor 36 of the HMD 30. Based on the target velocity value $P_{ref}$ overdot, a control-data generation section 44 generates control data CD and supplies the control data CD to the communication section 42 to operate the pneumatic cylinders 12, 18 and the vane motor 16 of the hold arm unit 10 so that the imager of the endoscope 24 follows the target velocity value.

The velocity-control computation section 48 performs computation in accordance with computation formulae to be described later, by following the computation steps shown in Expression 1.

Expression 1:

First, the velocity-control computation section 48 calculates an angular velocity instruction vector $\omega_{cmd}$ using the formula below, based on the set of signals GS representing the angular velocity vectors from the gyroscope 36.

Expression 2:

$$\omega_{\mathrm{cmd}} = K_r \cdot \omega_s$$

Kr is a velocity gain expressed in a matrix to be described later, and $\omega$s is an angular velocity vector. Specifically, $\omega$s is an angular velocity vector of the head obtained by the gyroscope, and if the angular velocity vector is subjected to a drift compensation (to be described later), $\omega$s is an angular velocity vector after the drift compensation.

The coordinate system used here is one fixed to the head. The center axis of the neck of the operator OP shown in Fig. 2 is the y axis, the lateral direction of the operator OP is the x axis, and the front-back direction of the operator OP is the z axis.

Further, multiplying the angular velocity by the constant Kr makes the sensitivity of movement set according to the user's preference. The constant Kr, may be set to different values for the respective directions. Kr may be a function.

Expression 3:

$$\boldsymbol{\omega}_s = \left(\omega_{sx}, \omega_{sy}, \omega_{sz}\right)^t$$

Expression 4:

$$K_r = \begin{bmatrix} K_{rx} & 0 & 0 \\ 0 & K_{ry} & 0 \\ 0 & 0 & K_{rz} \end{bmatrix}$$

Next, the velocity-control computation section 48 sets the angular velocity instruction vector $\omega_{cmd}$ to a predetermined limit value $\omega_{lim}$ through a limiter. To be more specific, if the angular velocity instruction vector $\omega_{cmd}$ exceeds the limit value $\omega_{lim}$, an angular velocity instruction vector $\omega'_{cmd}$ is set to the limit value $\omega_{lim}$, and if the angular velocity instruction vector $\omega_{cmd}$ is equal to or below the limit value $\omega_{lim}$, the angular velocity instruction vector $\omega'_{cmd}$ is set to the angular velocity instruction vector $\omega_{cmd}$. These settings keep the hold arm unit 10 from being excessively-fast operated, thereby preventing the imager from damaging an organ. Data on the value of the angular velocity instruction vector $\omega'_{cmd}$ is stored in the storage section 40M.

Next, in accordance with the formula shown below, the velocity-control computation section 48 obtains an angular velocity instruction vector $\omega''_{cmd}$ in an orthogonal coordinate system (Cx, Cy, Cz) of the distal end of the endoscope 24 (see Fig. 3) by transforming the angular velocity instruction vector $\omega'_{cmd}$ into local coordinates (Lx, Ly, Lx) of the hold arm (see Fig. 3) using a transformation matrix T, and multiplying the result by a matrix R. The orthogonal coordinate system has a coordinate axis Cz on the center axis of the inserted portion of the endoscope 24, i.e., directions in which the imager of the endoscope 24 advances or retreats.

Expression 5:

$$\boldsymbol{\omega}''_{cmd} = RT \cdot \boldsymbol{\omega}'_{cmd}$$

Expression 6:

$$R = E^{iq_1} E^{jq_2} E^{kq_4}$$

The matrix R represents the attitude of the endoscope 24, and is obtained sequentially through forward kinematics computation from joint displacements q of the hold arm unit 10 (see q1, q2, q4 in Fig. 3). E represents a rotation matrix.

Thereby, the upward, downward, leftward, and rightward directions in the screens of the display units of the HMD 30 always correspond to the upward, downward, leftward, and rightward directions of the head of the operator, respectively. In other words, the coordinate system fixed to the head wearing the HMD 30 corresponds to the coordinate system fixed to the distal end of the endoscope. Thus, images displayed on the display units of the HMD 30 follow the movement of the head of the operator.

In the example above, the velocity-control computation section 48 obtains the angular velocity instruction vector $\omega''_{cmd}$ in the orthogonal coordinate system (Cx, Cy, Cz) of the distal end of the endoscope 24 by transforming the angular velocity instruction vector $\omega'_{cmd}$ into local coordinates (Lx, Ly, Lx) of the hold arm using the transformation matrix T, and multiplying the result by the matrix R. However, the present invention is not limited to such an example. The transformation from the local coordinates (Lx, Ly, Lz) of the hold arm to the orthogonal coordinate system (Cx, Cy, Cz) of the distal end of the endoscope 24 may be omitted. For example, in a case where an image displayed on the display units of the HMD 30 is viewed externally as a CRT image, the transformation from the local coordinates (Lx, Ly, Lz) of the hold arm to the orthogonal coordinate system (Cx, Cy, Cz) of the distal end of the endoscope 24 may be omitted to enable superimposition of the CRT image and a CT image.

Next, in accordance with the formula shown below, the velocity-control computation section 48 transforms the angular velocity instruction vector $\omega''_{cmd}$ into a target velocity vector Vxy of the distal end (imager) of the endoscope 24. In other words, the angular velocity instruction vector is transformed into components Vxy in the upward, downward, leftward, and rightward directions of the target velocity of the distal end of the endoscope in the orthogonal coordinate system (Cx, Cy, Cz) by obtaining the cross product of the angular velocity instruction vector and a vector $l_3$ from the rotation center GP of the hold arm unit 10 to the distal end of the endoscope.

Expression 7:

$$v_{xy} = \omega''_{cmd} \times l_3$$

Next, the velocity-control computation section 48 performs computation on the target vector Vxy according to the formula below in order to adjust and change the velocity of the imager according to the amount of insertion of the imager of the endoscope into the body. Thereby, when the amount of insertion of the imager of the endoscope into the body increases, a target velocity vector V'xy of the imager of the endoscope is made larger. On the other hand, when the amount of insertion of the imager of the endoscope into the body decreases, i.e., when the imager of the endoscope is pulled out of the body, the target velocity vector V'xy of the imager of the endoscope is made smaller.

Expression 8:

$$v'_{xy} = \left(1 + r_{xy}q_3\right)v_{xy}$$

Multiplying the values of Vxy by a coefficient $r_{xy}$ depending on q3 (see Fig. 3) representing the amount of insertion of the distal end of the endoscope, as shown in the above formula adjusts the dependency of the amount of movement of the screen on the insertion level. This adjusts the amount of movement of a field of view by rotation of the head. For example, the amount of movement of a gaze object on the screen during rotating of the head is made almost constant irrespective of the zoom position. Hence, the operating intuition improves.

In the above formula, $r_{xy}$ is a constant and set within a range which does not reverse the sign of the value of Vxy. Note that, for q3, the direction for inserting the endoscope from a middle position is positive, and pulling it from the middle position is negative. The middle position is the center of a movable range of q3 in Fig. 3, and is set to zero. Note that $r_{xy}$ may be a function.

The velocity-control computation section 48 also calculates, in accordance with the formula below, a target velocity instruction vector Vz along the Cz coordinate axis (see Fig. 3) of the imager of the endoscope 24 based on a variable d determined based on the instruction signals Cz1 and Cz2 from the zoom-operation foot switch 52. The variable d is 1 if there is only the instruction signal Cz1, is -1 if there is only the instruction signal Cz2, and is 0 if there is neither one of the instruction signals Cz1 and Cz2.

Expression 9:

$$v_{\mathbf{z}} = (0, 0, K_z d)^t$$

In the above formula, Kz is a gain set by the user. Specifically, d=1 indicates zooming in, d=-1 indicates zooming out, and d=0 indicates no change.

Next, the velocity-control computation section 48 sets the target velocity instruction vector Vz to a predetermined limit value $V_{zlim}$ through a limiter. Specifically, if the target velocity instruction vector Vz exceeds the limit value $V_{zlim}$, a target velocity instruction vector V'z is set to the limit value $V_{zlim}$. If the target velocity instruction vector Vz is equal to or below the limit value $V_{zlim}$, the target velocity instruction vector V'z is set to the target velocity instruction vector Vz. These settings prevent excessively-fast operation of the hold arm unit 10. The hold arm unit 10 is thus prevented from operating excessively fast so that the endoscope does not touch and damage an organ. Safety is thereby ensured.

Next, the velocity-control computation section 48 transforms the obtained target velocity instruction vector V'z into a target velocity vector V"z of the distal end (the imager) of the endoscope 24 in accordance with the formula below. This makes the front-back movement of the head correspond to the front-back movement of the endoscope.

Expression 10:

$$v''_{\mathbf{z}} = RT \cdot v_{\mathbf{z}}'$$

The matrix R and the transformation matrix T are the same as those in the above expressions.

The velocity-control computation section 48 performs computation on the target velocity vector V"z in accordance with the formula below to adjust and change the velocity of the imager in response to the amount of insertion of the imager of the endoscope into the body.

Expression 11:

$$v'''_{\mathbf{z}} = (1 - r_z q_3) v''_{\mathbf{z}}$$

For the upward, downward, leftward, and rightward operations (movements of the rotations q1, q2 of the hold arm unit 10) (see Fig. 3), a gaze object is scaled up upon zooming in (deep insertion), and scaled down upon zooming out. As to the frontward and backward movements (movements of q3 of the hold arm for inserting the endoscope), reversed behaviors occur. Thereby, the extent of scaling up of the gaze object on the screen upon zooming in is made almost constant irrespective of the zoom position. In addition, while the endoscope is inserted deeply, the amount of zooming movement is made smaller, which avoids an unexpected contact between the endoscope and an organ.

Note that $r_z$ may be either one of a constant or a function.

Then, in accordance with the formula below, the velocity-control computation section 48 obtains the final target velocity value $P_{ref}$ overdot of the distal end (the imager) of the endoscope by adding upward, downward, leftward, and rightward velocity components and frontward and backward velocity component.

Expression 12:

$$\dot{P}_{\mathrm{ref}} = v'_{xy} + v'''_{\mathbf{z}}$$

In the above example, regarding a roll component (neck tilting) out of the rotation velocities of the head of the operator, a roll component of the above-described angular velocity instruction vector ω'cmd is directly given the target velocity of the roll q4 of the endoscope. However, the present invention is not limited to such an example. Moreover, such operation may be invalidated.

Although instructions for the frontward and backward directions are performed using the foot switch in the above description, the present invention is not limited to such a method. Other methods for generating frontward and backward instruction values use an acceleration sensor, an optical flow, skin displacement between the eyebrows, myoelectric potential measurement, or the like.

An advantageous effect offered by using the on/off-switching foot switch 50 is as follows. The switch is turned off when the operator does not want to move the endoscope. Then, the operator moves the head freely. In addition, for example, in a case where the operator moves the endoscope to the right with the switch on and reaches the rightward limit where the head is moved, the operator moves the endoscope to the right further by turning the switch off, bringing the head back to the left, turning the switch on again, and moving the head to the right. The endoscope does not work in conjunction with the movement of the head unless the switch is on, which avoids an unexpected operation.

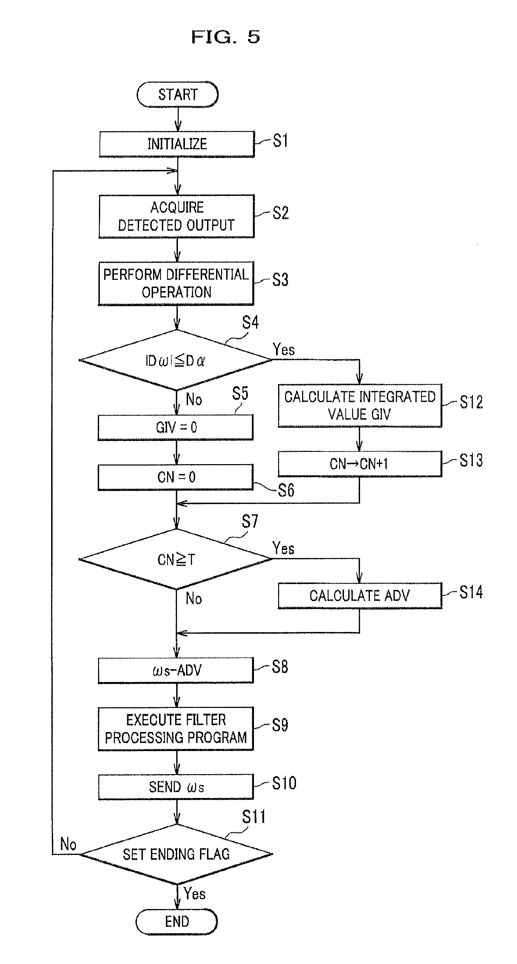

The drift-compensation computation section 46 of the control unit 40 is configured of, for example, a microcomputer. Programs executed by the microcomputer are described below with reference to a flowchart shown in Fig. 5.

In Fig. 5, after the flow starts, an offset value is set to a predetermined value ADV in Step S1. Then in Step S2, the set of signals GS and the set of signals EM are acquired. Signals GS represent the angular velocity vectors of the head of the operator in the above-described directions from the gyro sensor 36 of the HMD 30. Signals EM represent the tilt angles from each geomagnetic sensor 34 of the head of the operator in the above-described directions are acquired. In the subsequent Step S3, differential operation is performed based on the set of signals EM to obtain an angular velocity. Then, the flow proceeds to Step S4 where it is determined whether an absolute value |Dw| of the obtained angular velocity is equal to or below a threshold Dα or not. In other words, the drift-compensation computation section 46 determines whether the HMD 30 is stationary or not.

If it is determined in Step S4 that the absolute value |Dw| of the obtained angular velocity is not equal to or below the threshold Dα, i.e., the HMD 30 is not stationary, an integral GIV to be described later is set to zero in Step S5. Then, the flow proceeds to Step S6 to set a counter value CN of a stationary counter to zero. Then in Step S7, it is determined whether or not the counter value CN of the stationary counter is equal to or above a threshold T corresponding to a predetermined measurement time period.

If it is determined in Step S7 that the counter value CN of the stationary counter is below the threshold T, the flow proceeds to Step S8 to subtract the predetermined value ADV, being the offset value, from the angular velocity vector ωs which is based on the set of signals GS from the gyro sensor 36. The flow then proceeds to Step S9 to execute a filter processing program. Next, in Step S10, the angular velocity vector ωs is sent to the velocity-control computation section 48. The flow then proceeds to Step S11 to determine whether an ending flag is set or not. If an ending flag is set, the program is ended. If an ending flag is not set, the flow returns to Step S2. Then the steps of and following Step S2 are executed as described above.

If it is determined in Step S4 that the absolute value |Dw| of the obtained angular velocity is equal to or below the threshold Dα, i.e., the HMD 30 is stationary, the flow proceeds to Step S12 to calculate an integral GIV by integrating the angular velocity vector ωs based on the set of signals GS. Then in Step S13, the counter value CN of the stationary counter is incremented. The flow then proceeds to Step S7 to execute the steps of and following Step S7 as described above.

If it is determined in Step S7 that the counter value CN of the stationary counter is equal to or above the threshold T, the flow proceeds to Step S14 to divide the integral GIV by the counter value CN to calculate an average value ADV and store the average value ADV. Then, the flow proceeds to Step S8 to subtract the updated value ADV from the angular velocity vector ωs. The steps after that are executed as described above.

Thus, the drift-compensation computation section 46 of the control unit 40 solves the problem of zero-point drift caused by the set of signals GS from the gyro sensor 36. Hence, a situation is avoided where an image displayed on the display units 32 of the HMD 30 moves even though the head is not moving. In addition, a situation is avoided where the movement velocity (angular velocity) of an image varies depending on whether the head turns rightward or leftward. Further, position information on the HMD 30 is obtained more accurately, which enhances surgical safety.

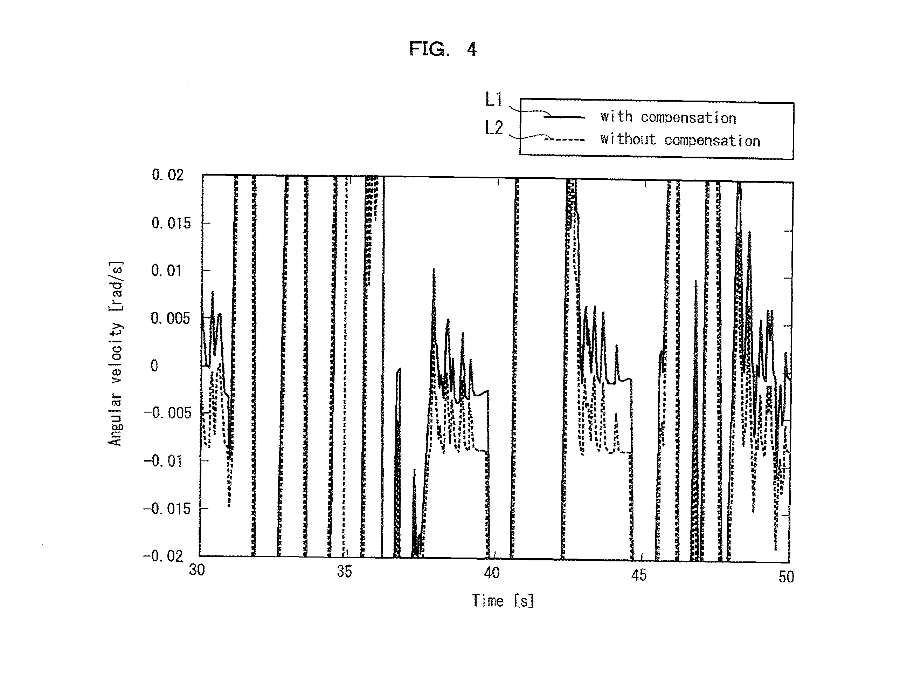

Fig. 4 shows characteristic lines indicating an effect obtained when the drift-compensation computation section 46 performs drift-compensation computation on an output value of an angular velocity based on the set of signals GS from the gyro sensor 36. In Fig. 4, the vertical axis indicates the angular velocity, and the horizontal axis indicates time. In addition, a characteristic line L1 indicates a result of performing the drift compensation computation, and a characteristic line L2 indicates a result of not performing the drift compensation computation.

As is apparent near 38 seconds in Fig. 4, the angular velocity indicated by the characteristic line L1 certainly converges near zero, compared to that indicated by the characteristic line L2.

It should be noted that the present invention is limited to the computation method shown in Expression 8. For example, any of the following computation methods may be employed. In addition, as the coefficient by which Vxy is multiplied, one which is 1 when q3 is 0 and which monotonically increases with q3 may be employed.

Expression 13:

$$v'_{xy} = \left(1 + r_{xy} q_3^n\right) v_{xy}$$

where n is an odd number

$$v'_{xy} = \exp\left(r_{xy} q_3\right) v_{xy}$$

$$v'_{xy} = \frac{1}{1 - r_{xy} q_3} v_{xy}$$

Moreover, the present invention is not limited to the computation method shown in Expression 11. For example, any of the following computation methods may be employed. In addition, as the coefficient by which V"z is multiplied, one which is 1 when q3 is 0 and which monotonically increases with q3 may be employed.

Expression 14:

$$v'''_{z} = \left(1 - r_z q_3^n\right) v''_{z}$$

where n is an odd number

$$v'''_{z} = \exp\left(-r_z q_3\right) v''_{z}$$

$$v'''_{z} = \frac{1}{1 + r_z q_3} v''_{z}$$

The movement velocity of the distal end of the endoscope may be controlled based on camera information on the imager of the endoscope. The camera information on the imager of the endoscope is a camera zoom factor.
The velocity-control computation section 48 performs computation on the target velocity vector Vxy to adjust and change the velocity of the imager according to the camera zoom factor. This enables adjustment of the amount of movement of a field of view due to rotation of the head. This enhances the operating intuition.

Moreover, the velocity-control computation section 48 performs computation on the target velocity vector V"z to adjust and change the velocity of the imager in response to the camera zoom factor. This enables adjustment of the amount of movement of a field of view due to frontward and backward movements of the endoscope. This enhances the operating intuition.

Moreover, in the above example, should the imager of the endoscope 24 touch an organ inside the body, the HMD 30 may inform the operator, and the present system may automatically shut down, from a safety perspective.

Further, although the coordinate system fixed to the head is transformed into local coordinates of the hold arm in the above example, the present invention is not limited to this method. The coordinate system may be taken in any way, so for example, the front-back direction of the head may be set to the y axis, and the vertical direction of the head may be set to the z axis. In addition, q1 and q2 may be calculated directly from the angular velocity of the head without the coordinate transformation, as in the case of the computation of q4.

In an example of the present invention, the following advantageous effects are offered.

1. Driving the pneumatic actuators such as pneumatic cylinders achieves passive soft operation, and prevents excessive driving force from being generated. The softness is easily achieved through pressure adjustment.

2. Should part of the endoscope touch an organ or the like, the force of the touching is enabled to be estimated based on a differential pressure of the pneumatic actuators.

3. It is designed such that a point of insertion into the abdominal wall mechanistically serves as a fixed point. A linear motion of the pneumatic cylinder is transformed into rotation by a slider crank mechanism so as to achieve vertical operations, and leftward and rightward operations and rotations are achieved by a pneumatic oscillating actuator. Frontward and backward operations are achieved for four degrees of freedom in total by using the linear motion of the pneumatic cylinder.

It should be noted that the present invention is not limited to the modes for carrying out the present invention described above, but may of course employ various other configurations without departing from the scope of the present invention.

EXPLANATION OF REFERENCE NUMERALS

10   hold arm unit
24   endoscope
30   HMD
34   geomagnetic sensor
36   gyro sensor
40   control unit
46   drift-compensation computation section
48   velocity-control computation section
52   zoom-operation foot switch
60   endoscope control system

**Claims**

1. An endoscope operation system comprising:

a hold arm unit reciprocatably and rotatably supported and configured to send attitudinal information thereof;
an endoscope supported by the hold arm unit and comprising an imager at a distal end thereof;
a display unit configured to display an image by a head mount display based on an image signal from the imager of the endoscope;
a posture detector comprising a sensor configured to detect a postural state of an operator; and
a foot switch,
wherein the distal end of the endoscope has a movement direction and a velocity to be controlled based on postural motion information from the posture detector, attitudinal information on the hold arm unit, and camera information on the imager of the endoscope.

2. The endoscope operation system according to claim 1,

wherein the posture detector configured to detect the postural state of the operator comprises a gyro sensor,
wherein the endoscope operation system further comprises a drift-compensation computation section configured to perform a compensation on an angular velocity vector which is based on a detection output,
wherein the drift-compensation computation section sends an angular velocity based on a postural displacement of the head of the operator,
wherein the attitudinal information on the hold arm unit comprises positional information on a hold arm to be sent, and
wherein the camera information on the imager of the endoscope comprises a camera zooming factor to be sent.

3. The endoscope operation system according to claim 2,
wherein the hold arm unit and the endoscope are driven by pneumatic actuators.

4. The endoscope operation system according to claim 2, further comprises a controller configured to perform a processing for computing a movement velocity of the endoscope based on: a value obtained by multiplying the angular velocity vector from the gyro sensor by a matrix representing an attitude of the hold arm unit and a transformation matrix; and the camera zooming factor from the imager.

5. The endoscope operation system according to claim 4,
wherein the controller for computing the movement velocity sets the movement velocity of the imager of the endoscope to a predetermined value or less.

6. The endoscope operation system according to claim 5,
wherein the controller for computing the movement velocity calculates a target velocity vector for the imager of the endoscope based on a variable indicated by an instruction signal from the foot switch.

7. The endoscope operation system according to claim 4,
wherein the controller adjusts a target velocity vector based on a coefficient representing an amount of insertion of the distal end of the endoscope.

8. The endoscope operation system according to claim 4,
wherein the controller is further connected to an on-off switching foot switch.

FIG. 1

FIG. 2

EP 2 856 923 A1

FIG. 3

FIG. 4

# FIG. 5

```
                    ┌─────────┐
                    │  START  │
                    └─────────┘
                         │
                         ▼
              ┌──────────────────────┐
              │     INITIALIZE       │──S1
              └──────────────────────┘
                         │
       ┌─────────────────┤
       │                 ▼
       │      ┌──────────────────────┐
       │      │       ACQUIRE        │──S2
       │      │   DETECTED OUTPUT    │
       │      └──────────────────────┘
       │                 │
       │                 ▼
       │      ┌──────────────────────┐
       │      │ PERFORM DIFFERENTIAL │──S3
       │      │     OPERATION        │
       │      └──────────────────────┘
       │                 │        S4
       │                 ▼
       │            ◇──────────◇         Yes
       │           ╱  |Dω|≦Dα   ╲───────────────┐
       │            ◇──────────◇                │
       │                 │ No    S5             ▼
       │                 ▼            ┌──────────────────────┐
       │      ┌──────────────────────┐│  CALCULATE INTEGRATED │──S12
       │      │       GIV = 0        ││     VALUE GIV         │
       │      └──────────────────────┘└──────────────────────┘
       │                 │                      │
       │                 ▼            ┌──────────────────────┐
       │      ┌──────────────────────┐│      CN→CN+1         │──S13
       │      │       CN = 0         ││                       │
       │      └──────────────────────┘└──────────────────────┘
       │             S6  │                      │
       │                 ◄──────────────────────┘
       │                 │        S7
       │                 ▼
       │            ◇──────────◇         Yes
       │           ╱   CN≧T     ╲───────────────┐
       │            ◇──────────◇                │
       │                 │ No                   ▼
       │                 │            ┌──────────────────────┐
       │                 │            │    CALCULATE ADV     │──S14
       │                 │            └──────────────────────┘
       │                 │                      │
       │                 ◄──────────────────────┘
       │                 ▼
       │      ┌──────────────────────┐
       │      │       ωs-ADV         │──S8
       │      └──────────────────────┘
       │                 │
       │                 ▼
       │      ┌──────────────────────┐
       │      │    EXECUTE FILTER    │──S9
       │      │  PROCESSING PROGRAM  │
       │      └──────────────────────┘
       │                 │
       │                 ▼
       │      ┌──────────────────────┐
       │      │       SEND ωs        │──S10
       │      └──────────────────────┘
       │                 │        S11
       │                 ▼
       │ No         ◇──────────◇
       └───────────╱ SET ENDING ╲
                    ╲   FLAG     ╱
                     ◇──────────◇
                         │ Yes
                         ▼
                    ┌─────────┐
                    │   END   │
                    └─────────┘
```

17

## INTERNATIONAL SEARCH REPORT

International application No.

PCT/JP2013/053772

### A. CLASSIFICATION OF SUBJECT MATTER
*A61B1/00*(2006.01)i, *A61B1/04*(2006.01)i

According to International Patent Classification (IPC) or to both national classification and IPC

### B. FIELDS SEARCHED

Minimum documentation searched (classification system followed by classification symbols)
A61B1/00, A61B1/04

Documentation searched other than minimum documentation to the extent that such documents are included in the fields searched
Jitsuyo Shinan Koho          1922-1996   Jitsuyo Shinan Toroku Koho   1996-2013
Kokai Jitsuyo Shinan Koho     1971-2013   Toroku Jitsuyo Shinan Koho   1994-2013

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
|---|---|---|
| Y | JP 2011-194163 A  (Olympus Corp.), 06 October 2011 (06.10.2011), paragraphs [0015], [0024], [0034], [0038], [0049], [0058], [0070] & WO 2011/118074 A1      & CN 102802553 A | 1-3 |
| Y | JP 8-332169 A  (Olympus Optical Co., Ltd.), 17 December 1996 (17.12.1996), paragraph [0123] & US 5836869 A | 1-3 |
| Y | JP 2001-145634 A  (Olympus Optical Co., Ltd.), 29 May 2001 (29.05.2001), paragraphs [0074], [0080] (Family: none) | 2,3 |

☒ Further documents are listed in the continuation of Box C.          ☐ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 08 March, 2013 (08.03.13) | 19 March, 2013 (19.03.13) |

| Name and mailing address of the ISA/ Japanese Patent Office | Authorized officer |
|---|---|
| Facsimile No. | Telephone No. |

Form PCT/ISA/210 (second sheet) (July 2009)

**INTERNATIONAL SEARCH REPORT**

| International application No. |
| --- |
| PCT/JP2013/053772 |

C (Continuation).   DOCUMENTS CONSIDERED TO BE RELEVANT

| Category* | Citation of document, with indication, where appropriate, of the relevant passages | Relevant to claim No. |
| --- | --- | --- |
| Y | JP 2009-285099 A  (Tokyo Institute of Technology),<br>10 December 2009 (10.12.2009),<br>paragraph [0014]<br>(Family: none) | 3 |
| A | JP 8-299363 A  (Olympus Optical Co., Ltd.),<br>19 November 1996 (19.11.1996),<br>paragraphs [0032], [0038]<br>(Family: none) | 1-8 |
| A | JP 9-28663 A  (Olympus Optical Co., Ltd.),<br>04 February 1997 (04.02.1997),<br>paragraphs [0076], [0079]<br>& US 5836869 A | 1-8 |
| A | JP 10-309258 A  (Olympus Optical Co., Ltd.),<br>24 November 1998 (24.11.1998),<br>paragraphs [0030], [0041], [0042]<br>(Family: none) | 1-8 |
| A | JP 2005-312919 A  (Toshiba Corp.),<br>10 November 2005 (10.11.2005),<br>fig. 27<br>& US 2005/222587 A1     & EP 1584300 A3 | 1-8 |
| A | JP 8-224245 A  (Olympus Optical Co., Ltd.),<br>03 September 1996 (03.09.1996),<br>paragraph [0028]<br>(Family: none) | 1-8 |
| A | JP 2005-312991 A  (Olympus Corp.),<br>10 November 2005 (10.11.2005),<br>paragraphs [0018], [0040]<br>(Family: none) | 1-8 |
| A | JP 9-66056 A  (Olympus Optical Co., Ltd.),<br>11 March 1997 (11.03.1997),<br>paragraphs [0017], [0018]<br>(Family: none) | 1-8 |
| A | JP 7-328016 A  (Olympus Optical Co., Ltd.),<br>19 December 1995 (19.12.1995),<br>paragraphs [0077], [0078]<br>& US 5876325 A | 1-8 |
| A | JP 2002-136471 A  (Olympus Optical Co., Ltd.),<br>14 May 2002 (14.05.2002),<br>abstract; paragraph [0031]<br>(Family: none) | 1-8 |

Form PCT/ISA/210 (continuation of second sheet) (July 2009)

**EP 2 856 923 A1**

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP HEI10309258 B